# EUROPEAN PATENT SPECIFICATION

(11) **EP 2 488 220 B1**
(45) Date of publication and mention of the grant of the patent: **29.07.2020**
(21) Application number: 10768275.9
(22) Date of filing: 18.10.2010
(51) Int. Cl.: A61L 9/12

(54) **AIR TREATMENT AGENT DISPENSER WITH IMPROVED ODOUR SENSOR FUNCTIONALITY**
SPENDER FÜR EINEN LUFTBEHANDLUNGSSTOFF MIT VERBESSERTER GERUCHSSENSORISCHER FUNKTION
DISTRIBUTEUR D'AGENT DE TRAITEMENT DE L'AIR COMPORTANT UNE FONCTIONNALITÉ AMÉLIORÉE DE DÉTECTION D'ODEURS

(30) Priority: 16.10.2009 GB 0918119
(43) Date of publication of application: 22.08.2012
(73) Proprietor: Reckitt & Colman (Overseas) Hygiene Home Limited, Slough, Berkshire SL1 3UH (GB)
(72) Inventor: AN, Yuan, Dongcheng District Beijing 10007 (CN); BLAGG, Adrian, Hull Yorkshire HU8 7DS (GB); MARRS, Paul, Hull Yorkshire HU8 7DS (GB); SOUTHERN, Louise, Hull Yorkshire HU8 7DS (GB); TOYNE, Suzanne, Hull Yorkshire HU8 7DS (GB); WITTY, Chris, Hull Yorkshire HU8 7DS (GB); WOOLLEY, Simon, Hull Yorkshire HU8 7DS (GB)
(74) Representative: Carlin, Robert George
(86) International application number: PCT/GB2010/051749
(87) International publication number: WO 2011/045617

(56) References cited:
- WO-A1-2004/037304
- WO-A2-2006/044416
- WO-A2-2008/068486
- WO-A2-2009/060212
- WO-A2-2009/090415
- JP-A- 2004 141 618

## Description

### Field of the Invention

The present invention relates to an emanation device that is configured to adapt the emanation of a fluid into the surrounding environment based on a determination of the characteristics of the surrounding environment and particularly, but not exclusively, for the emanation of air treatment agents such as fragrances, deodorizing and/or pest control materials.

### Background

Devices are known in which a bottle of volatile liquid has a upwardly projecting wick and a heater is located in the vicinity of the upper end of the wick to accelerate the evaporation of volatile liquid from the wick. The bottle, wick and heater are retained within a housing which carries an electric plug. To operate the heater the device is plugged into a wall socket. Devices of this type commonly claim to allow control of the rate of evaporation of the volatile liquids, for example, by varying the distance between the heater and the wick.

Devices are also known in which an aerosol air freshener is held within an automatic spraying device. A powered mechanism actuates the valve on the aerosol to periodically emit a spray of the air freshener. Devices of this type commonly claim to allow control of the amount of spray over a fixed time period by the consumer being able to vary the time period between emissions. Such automatic spraying devices are typically unable to provide adjustment in response to external stimuli.

Known prior art devices suffer from the drawback of efficiency and convenience. Typically the user has to manually change the apparatus from "normal" to "boost" mode, and then to switch it back to "normal" mode when this effect is no longer necessary (e.g. when the room is empty, or at night). Given the typical location of sources of electrical supply on walls (at a low level near the floor) or placement of electrical devices, this makes the switching process inefficient and inconvenient.

Timed devices are available which are configured to release air treatment agent at user-defined time periods but these device are not capable of dynamically adjusting their operation to take account of changes in the surrounding environment.

In order to overcome some of the drawbacks associated with a device having a timer function, devices consisting of a combination of a timed functional with a motion sensor functional have been made available and go some way to addressing this problem, however, increased motion surrounding the device does not necessarily linearly equate to a need for increased air treatment agent.

To improve the known devices yet further to make them truly adaptive to their surrounding environment it has been suggested that an odour sensor could be included with such devices such that when the device's 'electronic nose' detects malodour or the like air treatment agent could be emanated, however, such devices are fraught with sensitivity and reliability issues which renders them largely unsuitable for mass commercialisation.

WO 2009/060212 describes an emanation device comprising: a housing adapted to receive a container of fluid; sensor means provided by at least one motion sensor means and/or at least one odour sensor means; emanation means adapted to emanate, in use, the fluid; a controller in communication with said sensor means and said emanation means; wherein the controller is operative to: analyse the input from the sensor means; make a determination of the quantity of fluid to be emanated in response to said input; instruct the emanation means to emanate the determined quantity of fluid.

WO 2008/068486 describes a dispensing device for at least one air treatment agent comprising at least one airborne agent detector operable to detect airborne agents in the air; receiving means for receiving at least one source of air treatment agent; and control means for releasing an amount of at least one air treatment agent from a source thereof received in the receiving means when a current airborne agent level detected by the airborne agent detector deviates from a background airborne agent level detected by the airborne agent detector by more than a predetermined amount, wherein the background airborne agent level is calculated by the device.

WO 2009/090415 describes an emanation device comprising: a housing adapted to receive at least one container of fluid; emanation means adapted, in use, to emanate the fluid; a controller in communication with said emanation means; one or more remotely located sensor means in communication with the controller.

JP 2004-141618 describes a deodorizing aroma device for a toilet comprising at least a deodorizing function unit having a function of adsorbing or decomposing odours and a fragrance generating unit having a function of generating a fragrance, the fragrance generating unit is stopped during operation of the deodorizing function unit.

There is a need, therefore, for a device which overcomes the defects of the prior art.

### Summary of Invention

According to a first aspect of the present invention there is provided therefore a dispensing device for at least one air treatment agent, the dispensing device comprising:
a housing having one or more walls to define an interior adapted to receive at least one removable container of air treatment agent at least partially therein; and within said housing the device comprises:
an airborne agent detector means operable to detect airborne agents in the air, wherein said means are provided with at least one aperture to the exterior of the device to permit, in use, air from outside of the device to enter said airborne agent detector means;
receiving means for receiving said at least one container of air treatment agent;
emanation means adapted, in use, to emanate the air treatment agent from the device through one or more exit orifices in the housing;
control means in communication with the emanation means and in communication with said airborne agent detector means;
characterised in that said airborne agent detector means is substantially completely isolated from any fluid present in the interior of the housing such that any fluid present in the interior of the housing is substantially completely prevented from passing through said one or more housing walls to be detectable by the airborne agent detector means.

The housing preferably comprises a bottom wall, a top wall remote therefrom and one or more side walls therebetween. The one or more side walls preferably consist of a front wall, a rear wall opposed thereto and a left side wall and a right side wall between said front and rear walls. Most preferably the top wall is angled such it slopes downwardly toward the front wall. The exit orifice(s) is preferably provided in the top wall and/or in the side wall(s) at a position substantially adjacent to the top wall. In one most preferred arrangement the exit orifice(s) is provided in the sloping top wall such that air treatment agent emanated from the device, in use, is directed in a generally upward direction or a generally upward and forward direction perpendicular to the slope of the top wall. In an alternatively preferred arrangement the exit orifice(s) is provided in the front wall preferably located in a part of said front wall that is closer to the top wall than the bottom wall such that air treatment agent emanated from the device, in use, is directed in a generally forward direction at a height that may be suitable to permit the air treatment agent to be well dispersed in the surrounding environment.

Preferably the at least one aperture for the airborne agent detector means is spaced away from the one or more exit orifices. In one preferred arrangement the at least one aperture may be located in a housing wall that is substantially perpendicular with the housing wall of the exit orifice(s). In an alternatively preferred arrangement the at least one aperture may be located in a housing wall that is substantially opposite to the housing wall of the exit orifice(s). More preferably the at least one aperture is located in the side wall(s) of the housing and, even more preferably, is located in the rear wall.

The housing is preferably provided with a concave recess in a wall thereof that extends into the interior of the housing. The concave recess is preferably sized to receive the airborne agent detector means therein. The concave recess may be provided with a cover that is sized to fill the recess and to substantially follow the shape and/or contour of the side wall, and wherein said cover comprises the at least one aperture. In a preferred arrangement the airborne agent detector means is located within the recess and the cover is substantially permanently sealed to the adjacent side walls to substantially completely prevent ingress of any unwanted materials into the recess other than through the aperture(s) in the cover, and even more preferably completely prevent ingress of any unwanted materials into the recess other than through the aperture(s) in the cover.

Providing the airborne agent detector means within the recess in order to substantially completely isolate it from the interior of the housing has been found to be particularly advantageous insofar as the device may be better protected against false detections by said detector means due to the air treatment agent emanated by the device. With many known devices when the air treatment agent is emanated small quantities of the emanated agent are not successfully emanated into the surrounding environment but instead can get trapped within the device or impinge on the housing walls to circulate inside the device and/or pool inside the device and subsequently evaporate within the device. Without the isolation of the detector means the trapped emanated agent is able to interfere with, and in some cases completely saturate, the detector means such that it is rendered almost completely incapable of reading variations in airborne agents in the environment surrounding the device. Exposure to such high levels of detectable material, and particularly prolonged exposure, may also lead to problems associated with loss of sensitivity, detector means contamination, reduction of detector means lifetime and, ultimately, loss of detector means functionality.

The airborne agent detector means may be configured to wirelessly communicate with the control means in order to preserve the integrity of the recess against ingress of trapped emanated air treatment agent. Alternatively, a small conduit into the recess may be provided to permit a wired communication between the airborne agent detector means and the control means wherein any gaps between the conduit and the wire(s) is substantially completely sealed, and preferably completely sealed, to prevent the ingress of trapped emanated air treatment agent into the recess.

The aperture(s) may be provided with a filter membrane to prevent or substantially prevent particulate contamination of the airborne agent detector means whilst allowing gas diffusion therethrough. The filter membrane may be a plastics material with suitable diffusion properties such as a polyethylene membrane.

The device may be provided with a movable closure means which is configured to close the aperture(s) to the airborne agent detector means to the outside environment shortly before the device emanates an air treatment agent and, preferably said closure means remains closed for a period of time after emanation has occurred. At may be advantageous for the closure means to keep the aperture(s) closed for a period of time after emanation to allow the initial high concentration of air treatment agent surrounding the device immediately after emanation to subside as the agent emanates further into the surrounding environment in order to prevent false detections of airborne agent by the detector means. Preferably the closure means closes the aperture(s) between 1 to 60 seconds before emanation, and more preferably 1 to 30 seconds before emanation, and even more preferably 1 to 10 seconds before emanation. Preferably the closure means moves away from the aperture(s) to permit air from the surrounding environment to enter the aperture(s) between 1 second to 30 minutes after emanation, and more preferably 5 seconds to 15 minutes after emanation, and even more preferably 10 seconds to 10 minutes after emanation, and most preferably 15 seconds to 5 minutes after emanation.

The housing wall(s) may be provided with an outwardly extending protrusion adjacent to the aperture(s) communicating with the airborne agent detector means in order to prevent an emanated air treatment agent from entering the aperture(s) to falsely trigger the airborne agent detector means and/or potentially saturate the airborne agent detector means. Preferably the outwardly extending protrusion is provided in the form of a cowl located above the aperture(s) the divert any air treatment agent away from the aperture(s).

Preferably the airborne agent detector means comprises at least one odour sensor means. The odour sensor may comprise one or more metal oxide semiconductor sensors and/or one or more metal oxide sensors. The at least one odour sensor means may be combined with one or more additional sensors from the list of: a motion sensor; a person sensor; a light sensor; a sound sensor; a humidity sensor; a smoke sensor; a temperature sensor.

In order for any metal oxide semiconductor/metal oxide odour sensor to be operational the sensor must heat up to an operational temperature to facilitate suitably active surface chemistry on said sensor, typically this temperature is in the order of 300 to 380°C. The need to access such high operation temperatures makes devices containing such sensors consume large amounts of energy. It is a further object of the present invention to improve the energy consumption of devices containing oxide semiconductor/metal oxide odour sensor(s) and to this end the inventors have realised that it is possible to achieve this aim via pulsing the energy applied to said sensor(s).

In one preferred arrangement power is applied to the sensor(s) substantially continuously in order to get the sensor(s) to an operational temperature to ensure that said sensor(s) surface chemistry is suitably active to detect airborne agents and thereafter the power is applied intermittently to the sensor(s) to keep the sensor(s) at or close to an operational temperature and/or to ensure that said sensor(s) surface chemistry is suitably active to detect airborne agents.

In another preferred embodiment after the power has been applied to the sensor(s) substantially continuously to get the sensor(s) to an operational temperature to ensure that said sensor(s) surface chemistry is suitably active to detect airborne agents, thereafter the power is applied intermittently to the sensor(s) to keep the sensor(s) at an operational temperature and/or to ensure that said sensor(s) surface chemistry is suitably active to detect airborne agents such that the odour sensor may substantially continuously or routinely measure the to quantities of airborne agents entering the aperture(s).

In an alternatively preferred embodiment after the power has been applied to the sensor(s) substantially continuously to get the sensor(s) to an operational temperature to ensure that said sensor(s) surface chemistry is suitably active to detect airborne agents, thereafter the power is applied generally intermittently to the sensor(s) to keep the sensor(s) close to an operational temperature and/or to ensure that said sensor(s) surface chemistry is suitably active to detect airborne agents. Within the period of intermittent power application may be periods of continual power application and/or increased power application to temporally place the sensor(s) at the operation temperature and/or to ensure that said sensor(s) surface chemistry is suitably active to detect airborne agents, wherein the odour sensor is arranged to cooperate with this application of power thereto to only measure the quantities of airborne agents entering the aperture(s) when the sensor(s) is at the operational temperature and/or to ensure that said sensor(s) surface chemistry is suitably active to detect airborne agents in the power application cycle.

In a further alternatively preferred embodiment after the power has been applied to the sensor(s) substantially continuously to get the sensor(s) to an operational temperature to ensure that said sensor(s) surface chemistry is suitably active to detect airborne agents, thereafter follows a period of no application of power, wherein the non-application of power is followed by the application of power substantially continually to get the sensor(s) to an operational temperature to ensure that said sensor(s) surface chemistry is suitably active to detect airborne agents, and the cycle continues in accordance with the same pattern throughout the operation of the device.

Preferably the once the metal oxide semiconductor/metal oxide odour sensor to has been heated up to an operational temperature the pulses of power may last for a 5 to 1000ms period with an off-period lasting between 0.5 to 10 seconds, and more preferably the pulses of power last for a 5 to 250ms period with an off-period lasting between 0.5 to 7.5 seconds, and even more preferably the pulses of power last for a 5 to 100ms period with an off-period lasting between 0.5 to 5.5 seconds, and most preferably the pulses of power last for a 5 to 60ms period with an off-period lasting between 0.5 to 3.5 seconds, ideally the pulses of power last for substantially 35ms with an off-period lasting for substantially 2.2 seconds.

In the context of the present invention and for the avoidance of doubt "operational temperature" is used in relation to the present invention to relate to the temperatures that the sensor(s) must access to facilitate suitably active surface chemistry on said sensor. For the further avoidance of doubt, in the context of the present invention "close to an operational temperature" is understood to mean the temperature is only permitted to drop below an operation temperature defined by the duration of time (as defined above) that it would take the device under the application of power to the sensor to heat up to an operational temperature. The skilled person would understand the boundaries of "close" to be based on how the system had been tuned when the sensor(s) of the device may be powered 5 to 1000ms period with an off-period lasting between 0.5 to 10 seconds; such that a temperature would not be defined as "close to an operational temperature" if the sensor was not capable of reaching an operational temperature within the operational boundaries the device was tuned to.

In order to maintain and/or improve the sensitivity of the airborne agent detector means and prevent the false triggering thereof, once a quantity of air treatment agent has been emanated the control means may prevent the airborne detection means from operating for a period of time to allow the initial high concentration of air treatment agent surrounding the device immediately after emanation to subside as the agent emanates further into the surrounding environment in order to prevent false detections of airborne agent by the detector means. Preferably the control means is operable to disable the airborne agent detector means from operating for between 1 second to 30 minutes after emanation, and more preferably 5 seconds to 15 minutes after emanation, and even more preferably 10 seconds to 10 minutes after emanation, and most preferably 15 seconds to 5 minutes after emanation, and ideally for substantially between 90 to 180 seconds after emanation; by virtue of this arrangement the airborne agent detector will also be conserving power consumption which is particularly useful if the device is to be non-mains electric powered. Alternatively the control means may be operable to ignore and/or dismiss signals from the airborne agent detector means for between 1 second to 30 minutes after emanation, and more preferably 5 seconds to 15 minutes after emanation, and even more preferably 10 seconds to 10 minutes after emanation, and most preferably 15 seconds to 5 minutes after emanation, and ideally for substantially between 90 to 180 seconds after emanation.

In order to further maintain and/or improve the sensitivity of the airborne agent detector means the control means may be provided with auto-ranging functionality whereby said functionality is operable to select the correct range of the signals received from the airborne agent detector in order to ensure consistent response is maintained to further airborne agents whilst the detector means is already under the influence of previously existing airborne agent(s) or other environmental factors.

Preferably the at least one airborne agent detector operable, in use, to detect whether the current airborne agent level deviates from a background airborne agent level detected by more than a predetermined amount, wherein the background airborne agent level and the current airborne agent level is calculated by the device, preferably by the control means.

Preferably, the control means are operable to calculate the current airborne agent level by calculating an average of a predetermined number of most recent readings of the airborne agent detector means. Preferably, two to five of the most recent readings, more preferably three of the most recent readings.

The control means may be operable to calculate the deviation of the current airborne agent level from the background level by means of a subtraction of one from the other, and/or by means of a ratio of one to the other.

Preferably, the deviation is calculated by subtracting the background level from the current airborne agent level and dividing that amount by the background level value. The result may be multiplied by a constant, for ease of display and/or use.

Unlike several known prior art devices the device of the present invention does not operate using a pre-defined value for the background airborne agent level, rather the device of the present invention calculates this level and uses this calculated level to control the release of the at least one air treatment agent. This arrangement may be advantageous as the device is operable to adapt how it releases the one or more air treatment agent depending on the characteristics of the surrounding environment in which it is used.

Preferably, the control means are operable to calculate the background airborne agent level by calculating an average of a longer time period than that over which the current airborne agent level is calculated.

Preferably, the background airborne agent level and the current airborne agent level are temporally offset, preferably by at least 5 seconds, more preferably by at least 10 second, more preferably by at least 20 seconds.

Once the device is placed into an operational mode, the background airborne agent level may be an average of the levels of airborne agent detected by the device throughout the duration of that operational mode. In this arrangement the device may better 'learn' the characteristics of its local environment and, during use, will be better able to provide for the release of an air treatment agent(s) when the current airborne agent level deviates from the background level by more than the predetermined amount. If a user wishes to move the device to an alternative location, a user may be encouraged to reset the device from the operational mode, this resetting of the device may have the effect of zeroing the average levels of background agent such that the device is operable to 'learn' the characteristics of its new environment when placed back into the operational mode by calculating the average background agent level from no existing starting point.

Preferably, the control means are operable to calculate the background airborne agent level by calculating an average of a predetermined number of some or all of the most recent readings of the detector. Preferably 10 to 10,000 of the most recent readings, more preferably 20 to 5,000 of the most recent readings, and most preferably 50 to 1000 of the most recent readings.

The device may be provided with an initial setting mode wherein when the device is first powered up, the control means will automatically calibrate based on the existing background odour when the device is first switched on.

In an alternative or additional arrangement, the control means are preferably operable to calculate the background level based on calculating a series of averages from rolling windows of measurements from the airborne agent detector means. Each rolling window may be an average of between two and ten readings, preferably six readings. Preferably, the windows do not overlap. Preferably, the windows span a time period of between 5 and 30 minutes, preferably between 10 and 25 minutes, preferably between 15 and 20 minutes. There may be approximately 30 to 50 windows.

Preferably, the control means are operable to discard the oldest window when a new window becomes available, preferably taking into account an offset between the current and background levels.

Preferably, the control means are operable to adjust the predetermined level of deviation from the background level that results in air treatment agent being released. The predetermined level may be manually adjustable. The deviation may be a positive or negative deviation.

Where the airborne agent detector means is provided in the form of one or more metal oxide semiconductor/metal oxide odour sensor said sensor(s) may be provided with one or more resistors in series therewith to ensure a consistent output of signal from the sensor(s) as their resistance changes during their operation in response to their detection of airborne agent. Preferably the device is provided with between 3 to 5 dynamic range resistors with a 1 to 300KΩ range.

The emanation means could be provided by one or more known emanation mechanisms for air treatment agents. For instance, where the air treatment agent is contained in a pressurised container with a suitable propellant (such as hydrocarbon propellant, compressed gas, compressed nitrogen, or the like) the emanation mechanism may be the mechanical movement of the container's valve either by direct contact with the valve or by movement of the valve actuator; in this arrangement the container may be a metered dose aerosol to improve the spray performance and reproducibility thereof. Alternatively the pressurised container could be engaged in the device with its valve held open and a electro-magnetic solenoid is employed to control the release of the air treatment agent. Other suitable emanation means may include, but is not necessarily limited to, one or more heaters, one or more nebulisers, one or more piezoelectric emanation means.

The device is preferably provided with one exit orifice per replaceable container of air treatment agent secured in the device, this arrangement is preferably to prevent cross-contamination of the air treatment agents.

Preferably the container of air treatment agent is received entirely within the housing of the device.

The device may be provided with a user-controlled boost mechanism. In use of the device, the activation of said boost mechanism may substantially immediately cause the dispensing of the at least one air treatment agent.

The airborne agents detected by the airborne agent detector means may be common household odours (and the chemicals which constitute) these malodours. For example: kitchen malodour; bathroom malodour; tobacco smoke; pet odours; mould and/or mildew; body odour; fish; onions; garbage; fragrance from other products (such as detergents, polishes, cleaning products etc). To facilitate such detection the odour sensor means may be operable to detect at least some of the following chemical components: amines and nitrogen compounds; acids and/or sulphur compounds, such as mercaptans, thioacids, thioesters, sulfides, phenols and skatole.odours..

The device of any of the above-mentioned aspects may be provided with an indicator wherein said indicator is operable to indicate to a user what function the device is currently performing. The indicator may be operable to provide a visual indication and/or provide an audible indication.

Preferably the indicator is configured to provide a visual indication by emitting light from one or more light sources, preferably one or more LEDs.

The one or more light sources may be adapted to emit a different colour of light to indicate the current function the device is performing. Additionally or alternatively, the one or more light sources may blink or flash to indicate the current function the device is performing.

Alternatively or additionally, the device may be operable to visually indicate the function currently being performed by the device via a screen. The screen may be an LCD screen that is adapted to provide a message to a user, for instance such messages could include "ON", "SENSING", "MOTION DETECTED", "RESTING", "NORMAL MODE", "DETECTION MODE", "OFF".

The device may be power by mains-supplied electricity and/or be battery powered and/or be powered by solar cells located on the device. Most preferably the device is battery powered.

According to a second aspect of the present invention there is provided therefore a method of improving the sensitivity of a dispensing device with an airborne agent detector mean for at least one air treatment agent, wherein the dispensing device comprises:
a housing having one or more walls to define an interior adapted to receive at least one removable container of air treatment agent at least partially therein; and within said housing the device comprises:
   an airborne agent detector means operable to detect airborne agents in the air, wherein said means are provided with at least one aperture to the exterior of the device to permit, in use, air from outside of the device to enter said airborne agent detector means;
   receiving means for receiving said at least one container of air treatment agent;
   emanation means adapted, in use, to emanate the air treatment agent from the device through one or more exit orifices in the housing;
   control means in communication with the emanation means and in communication with said airborne agent detector means;
   and wherein said airborne agent detector means is substantially completely isolated from any fluid present in the interior of the housing such that any fluid present in the interior of the housing is substantially completely prevented from passing through said one or more housing walls to be detectable by the airborne agent detector means;
and wherein the method comprises the steps of:
   loading a container of air treatment agent into said housing and engaging the receiving means; placing the device in an operational mode wherein the emanation means engage with the container of air treatment agent to cause a quantity of air treatment agent to emanate from the device;
   characterised in that any air treatment agent that remains in the device after the emanation thereof is not capable to emanate from within the device to come into contact with the airborne agent detector which is substantially completely isolated therefrom.

Any of the features described herein may be combined with any of the above aspects in any combination.

### Description of an Embodiment

Embodiments of the invention will now be described, by way of example only, with reference to the following drawings in which:
Fig. 1 illustrates a cross sectional view of an autospray device according to the present invention;
Fig. 2 illustrates a rear elevation of an autospray device according to the present invention.
Fig. 3 illustrates a front elevation of a plug-in electrical device according to the present invention;
Fig. 4 illustrates a top elevation of the plug-in electrical device according to the present invention;
Fig. 5 illustrates a cross sectional view of the plug-in electrical device according to the present invention; and
Fig. 6 illustrates a side elevation of a plug-in electrical device according to the present invention;

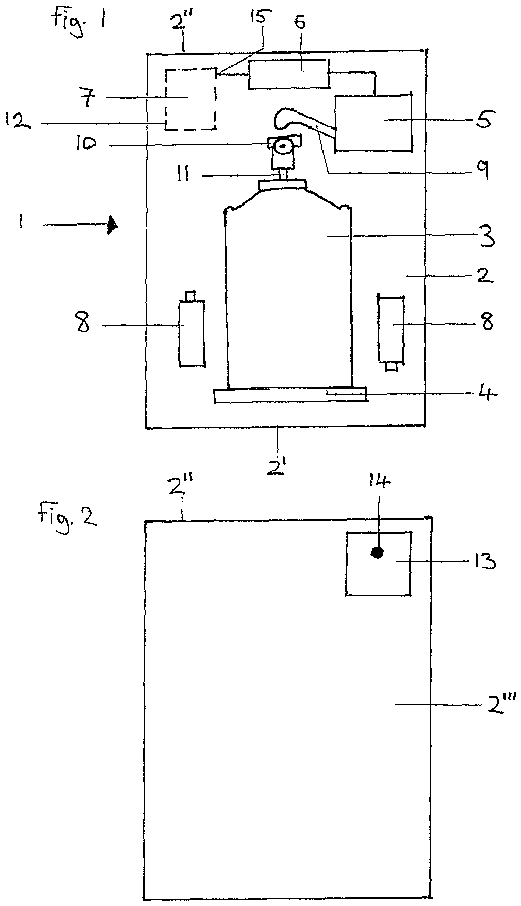

Fig.1 illustrates a cross-sectional view of an autospray device 1 for the emanation of air treatment agent according to the present invention. The device 1 comprises a housing 2 and Fig. 1, in essence, shows the housing absent the front wall of the housing 2. The front wall of the housing is provided with one or more exit orifices (not shown). The housing is sized to receive a removable container 3 of air treatment agent therein. In Fig. 1 the container 2 of air treatment agent is provided in the form of an aerosol canister containing air treatment agent under pressure, the canister sits on receiving means 4 which in this embodiment are provided in the form of a platform that supports the base of the aerosol. Also illustrated in Fig.1 are the emanation means 5, the control means 6, airborne agent detector means 7 and a power source 8 which is Fig.1 are depicted as a pair of batteries, and these will all be discussed in great detail below.

The emanation means 5 is provided with an arm 9 that is movable between at least two positions, the first of these positions is illustrated in Fig.1 in which the arm is in a raised position above the aerosol canister. The aerosol canister has a valve connected to an actuator 10 via a valve stem 11 therebetween. In the first position the arm 9 is held at least partially above the actuator 10 and the aerosol valve remains in a closed position. In the second position of the arm 9, the emanation means 5 causes the arm to move in a substantially downward direction to depress the actuator 10 toward the valve, the valve stem 11 is depressed and the valve opens to permit air treatment agent to exit from the aerosol canister. Preferably the container 3 is a metered dose aerosol canister which may be advantageous as a single depression of the spray head will release a predefined quantity of fluid from the aerosol canister regardless of the duration of time the actuator is depressed. However, a non-metered dose aerosol may be used in the device 1 as could a non-pressurised container possessing a pump mechanism to spray the air treatment agent therefrom.

Alternatively, the emanation means 5 could take the form of a valve system, such as a solenoid valve system (not shown). Such a solenoid valve system may work together with a pressurised aerosol engaged therewith. Rather than initiate actuation by movement, the solenoid valve would be energised to initiate the release of a quantity of fluid from the aerosol.

Although not illustrated, the device 1 may be provided with means to receive at least two separate containers of air treatment agent. In this arrangement the device 1 may be provided with additional emanation means to cause the emanation of the agent, or a single set of emanation means 5 to emanate agent from both containers as directed by the control means 6.

The control means 6 is operationally connected to the airborne agent detector means 7 and the emanation means 5 such that it is able to communicate therebetween.

The airborne agent detector means 7 is shown to be located within dotted lines, that is because in Fig.1 the detector means 7 is substantially completely isolated from the interior of the housing such that any fluid present in the interior of the housing is substantially completely prevented from passing through the housing walls 2 to be detectable by the detector means 7.

The housing 2 comprises a bottom wall 2', a top wall 2", a front wall (not shown) and a rear wall 2'''. The rear wall 2'" is provided with a recess 12 that extends into the interior of the housing 2, the recess 12 being concave in shape when the device is viewed from the angle depicted in Fig.2. The recess 12 is sized to receive the airborne agent detector means 7 therein. The recess is provided with a cover 13 that is sized to fill the recess to and substantially follow the shape and/or contour of the exterior-facing rear wall 2'''. The cover 13 is provided with at least one aperture 14 to permit air outside of the device 1 to enter and its content be analysed by the detector means 7. As shown in Figs. 1& 2, the airborne agent detector means 7 is located within the recess 12 and the cover 13 is substantially permanently sealed to the adjacent rear wall 2''' to substantially completely prevent ingress of any unwanted materials into the recess other than through the aperture 14 in the cover 13.

The arrangement shown in Figs. 1 & 2 illustrates how the airborne agent detector means 7 is substantially completely isolated from the interior of the housing and this is considered to be advantageous insofar as the device may be better protected against false detections by said detector means 7 due to the air treatment agent erroneously and/or routinely being emanated by the device within the interior of the housing 2. A small conduit 15 into the recess is provided to permit a wired communication between the airborne agent detector means 7 and the control means 6, the gap between the conduit 15 and the wire(s) is sealed to prevent the ingress of trapped emanated air treatment agent into the recess such as with resin or adhesive or the like.

Although not illustrated, the airborne agent detector means 7 could wirelessly communicate with the control means 6 in order to preserve the integrity of recess against ingress of air treatment agent present in the interior of the housing 2.

Although not illustrated the aperture 14 can be filled with a filter membrane to prevent or substantially prevent particulate contamination of the airborne agent detector means whilst allowing gas diffusion therethrough. The filter membrane may be a plastics material with suitable diffusion properties such as a poly ethylene membrane.

It can be seen in Fig.1 that the actuator 10 of the aerosol canister is arranged to spray air treatment agent in a forward direction through the front wall of the housing (not shown) at an angle that is generally parallel or at a slightly elevated angle to the bottom wall 2' of the housing, typically the device 1 will stand on a surface with its bottom wall 2' in contact with said surface. This arrangement ensures that the aperture 14 is spaced away from the exit orifice, and in the arrangement illustrated the aperture 14 is located in a housing wall that is substantially opposite to the housing wall of the exit orifice such that air treatment agent sprayed from the container 2 will not immediately come into contact with the aperture 14.

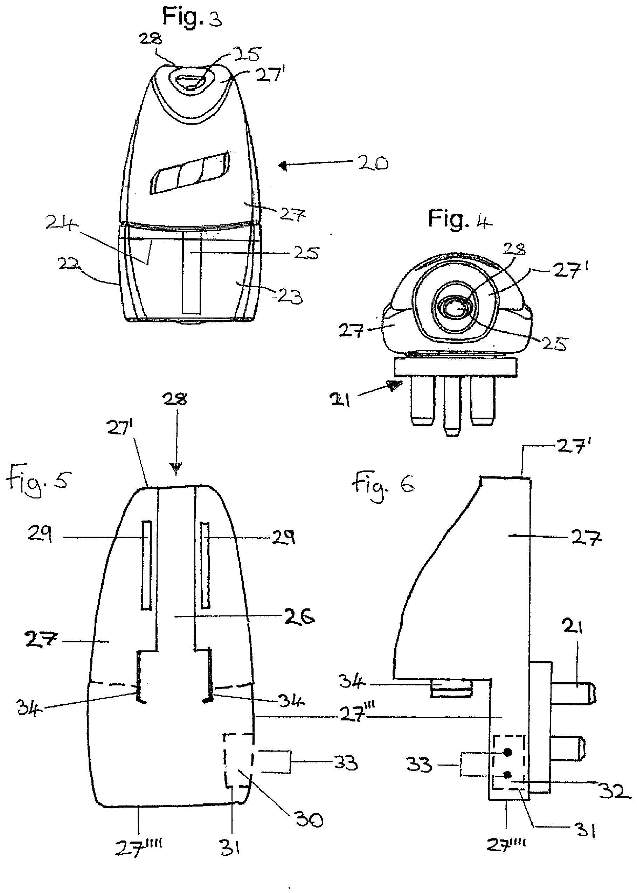

Turning now to Figs. 3-6 the device 20 is a plug-in device intended to connected to a mains electricity socket, the device being mounted on or carried by a electrical plug formations 21 that extend out of the rear side of the device 20. The device 20 is illustrated in Figs. 3 & 4 with a container 22 of volatile liquid air treatment agent engaged therewith, held in place by receiving means 34. The container 22 has a reservoir portion 23 in the form of a glass bottle containing the air treatment agent 24 and a wick 25 extending from the reservoir 23 to above the top of the bottle through a seal (not shown) and into a chimney means 26 of the device 20. The wick 25 may be substantially cylindrical and the seal is present to retain the air treatment agent 24 within the bottle should the device 20 be knocked over and/or inverted when the container 22 is engaged therewith.

The device 20 has a housing 27 which partially extends over an upper part of the container 22. The top of the housing 27 has a generally circular central exit orifice 28 which is aligned with the intended airflow from the chimney means 26.

The emanation means may be provided in the form of at least one heater means 29 and/or at least one electric fan (not shown). The heating means 29 are illustrated as resistors, such as positive temperature coefficient (PTC) thermistors but said means could be provided by way of a ring heater or the like, or a combination thereof.

Although not illustrated, the device 20 may be provided with means to receive at least two separate containers of liquid 22. In this arrangement the device 20 may be provided with additional emanation means to cause the emanation of the air treatment agent 24.

Most preferably the top wall 27' of the housing 27 is angled such it slopes downwardly toward the front wall 27" and the exit orifice 28 is provided in the top wall 27'. In use, to be discussed in more detail below, air treatment agent 24 emanated from the device 20 is directed in a generally upward direction or a generally upward and forward direction perpendicular to the slope of the top wall 27'.

The airborne agent detector means 30 is provided in the side wall 27''' generally adjacent the bottom wall 27"" of the housing 27 located within dotted lines as said detector means 30 is substantially completely isolated from the interior of the housing such that any fluid present in the interior of the housing is substantially completely prevented from passing through the housing walls to be detectable by the detector means 30.

The housing 27 comprises is provided with a recess 31 that extends into the interior of the housing 27, the recess 31 being concave in shape when the device is viewed from the angle depicted in Fig.6. The recess 31 is sized to receive the airborne agent detector means 30 therein. The recess is provided with a cover 32 that is sized to fill the recess to and substantially follow the shape and/or contour of the exterior-facing side wall 27'''. The cover 32 is provided with at least one aperture 33 to permit air outside of the device 20 to enter and its content be analysed by the detector means 30. The airborne agent detector means 30 is located within the recess 31 and the cover 32 is substantially permanently sealed to the adjacent side wall 27''' to substantially completely prevent ingress of any unwanted materials into the recess other than through the aperture 33 in the cover 32.

As with the arrangement shown in Figs. 1 & 2, device 20 is arranged such that the airborne agent detector means 30 is substantially completely isolated from the interior of the housing and this is considered to be advantageous insofar as the device may be better protected against false detections by said detector means 30 due to the air treatment agent erroneously and/or routinely being emanated by the device 20 within the interior of the housing. A small conduit (not shown) into the recess is provided to permit a wired communication between the airborne agent detector means 30 and a control means (not shown), the gap between the conduit and the wire(s) is sealed to prevent the ingress of trapped emanated air treatment agent into the recess such as with resin or adhesive or the like. Although not illustrated, the airborne agent detector means 30 could wirelessly communicate with the control means in order to preserve the integrity of recess 31 against ingress of air treatment agent 24 present in the interior of the housing.

Although not illustrated the aperture 33 can be filled with a filter membrane to prevent or substantially prevent particulate contamination of the airborne agent detector means whilst allowing gas diffusion therethrough. The filter membrane may be a plastics material with suitable diffusion properties such as a poly ethylene membrane.

For both devices 1, 20 illustrated, the air borne agent detector means 7, 30 generally comprises at least one odour sensor means, and preferably one or more metal oxide semiconductor sensors and/or one or more metal oxide sensors.

The mode of operation of the devices 1, 20 and the inter-relation of the components will now be explained.

In order for any metal oxide semiconductor/metal oxide odour sensor to be operational the sensor must heat up to an operational temperature, typically this temperature is in the order of 300 to 350°C. The devices 1, 20 may be arranged such that their control means allows power to be applied to the sensor(s) substantially continuously in order to get the sensor(s) to an operational temperature and thereafter the power is applied intermittently to the sensor(s) to keep the sensor(s) at, or close to, an operational temperature such that the odour sensor may substantially continuously or routinely measure the to quantities of airborne agents entering the aperture14, 33.

Once the metal oxide semiconductor/metal oxide odour sensor to has been heated up to an operational temperature the pulses of power may last for a 5 to 1000ms period with an off-period lasting between 0.5 to 10 seconds, and more preferably the pulses of power last for a 5 to 250ms period with an off-period lasting between 0.5 to 7.5 seconds, and even more preferably the pulses of power last for a 5 to 100ms period with an off-period lasting between 0.5 to 5.5 seconds, and most preferably the pulses of power last for a 5 to 60ms period with an off-period lasting between 0.5 to 7.5 seconds, ideally the pulses of power last for substantially 35ms with an off-period lasting for substantially 2.2 seconds.

To improve and/or maintain the sensitivity of the airborne agent detector means 7, 30 and prevent the false triggering thereof, once a quantity of air treatment agent has been emanated the control means prevents the airborne detection means 7, 30 from operating for a period of time to allow the initial high concentration of air treatment agent surrounding the device immediately after emanation to subside as the agent emanates further into the surrounding environment in order to prevent false detections of airborne agent by the detector means 7, 30. Preferably the control means is operable to disable the airborne agent detector means 7, 30 from operating for between 1 second to 30 minutes after emanation, and more preferably 5 seconds to 15 minutes after emanation, and even more preferably 10 seconds to 10 minutes after emanation, and most preferably 15 seconds to 5 minutes after emanation, and ideally for substantially 100 seconds after emanation; by virtue of this arrangement the airborne agent detector 7, 30 will also be conserving power consumption which is particularly useful if the device depicted in Figs.1 & 2 which may be non-mains electric powered.

In use the control means is arranged to analyse signals received from the airborne agent detector means 7, 30 to detect whether the current airborne agent level deviates from a background airborne agent level detected by more than a predetermined amount, wherein the background airborne agent level and the current airborne agent level is calculated by said control means.

The control means are operable to calculate the current airborne agent level by calculating an average of a predetermined number of most recent readings of the airborne agent detector means 7, 30. Preferably, two to five of the most recent readings, more preferably three of the most recent readings.

The control means may be operable to calculate the deviation of the current airborne agent level from the background level by means of a subtraction of one from the other, and/or by means of a ratio of one to the other.

Preferably however, the deviation is calculated by subtracting the background level from the current airborne agent level and dividing that amount by the background level value. The result may be multiplied by a constant, for ease of display and/or use.

The control means are operable to calculate the background airborne agent level by calculating an average of a longer time period than that over which the current airborne agent level is calculated. To improve the sensitivity the background airborne agent level and the current airborne agent level are temporally offset, preferably by at least 5 seconds, more preferably by at least 10 second, more preferably by at least 20 seconds.

Once the device 1, 20 is placed into an operational mode, the background airborne agent level may be an average of the levels of airborne agent detected by the device throughout the duration of that operational mode. In this arrangement the device 1, 20 may better 'learn' the characteristics of its local environment and, during use, will be better able to provide for the release of an air treatment agent(s) when the current airborne agent level deviates from the background level by more than the predetermined amount.

If a user wishes to move the device 1, 20 to an alternative location, a user may be encouraged to reset the device from the operational mode, this resetting of the device may have the effect of zeroing the average levels of background agent such that the device is operable to 'learn' the characteristics of its new environment when placed back into the operational mode by calculating the average background agent level from no existing starting point.

The control means are operable to calculate the background airborne agent level by calculating an average of a predetermined number of some or all of the most recent readings of the detector, preferably 10 to 10,000 of the most recent readings.

The device 1, 20 may be provided with an initial setting mode wherein when the device is first powered up, the control means will automatically calibrate based on the existing background odour when the device is first switched on. Thereafter the control means are operable to calculate the background level based on calculating a series of averages from rolling windows of measurements from the airborne agent detector means. Each rolling window may be an average of between two and ten readings, preferably six readings. Preferably, the windows do not overlap. Preferably, the windows span a time period of between 5 and 30 minutes, preferably between 10 and 25 minutes, preferably between 15 and 20 minutes. There may be approximately 30 to 50 windows. The control means are operable to discard the oldest window when a new window becomes available, preferably taking into account an offset between the current and background levels.

Preferably, the control means are operable to adjust the predetermined level of deviation from the background level that results in air treatment agent being released. The predetermined level may be manually adjustable. The deviation may be a positive or negative deviation.

Where the airborne agent detector means is provided in the form of one or more metal oxide semiconductor/metal oxide odour sensor said sensor(s) may be provided with one or more resistors in series therewith to ensure a consistent output of signal from the sensor(s) as their resistance changes during their operation in response to their detection of airborne agent. Preferably the device is provided with between 3 to 5 dynamic range resistors with a 1 to 300KΩ range.

The device 1, 20 may be provided with a user-controlled boost mechanism (not shown). In use of the device, the activation of said boost mechanism may substantially immediately cause the dispensing of the at least one air treatment agent.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying abstract and drawings) may be replaced by alternative features serving the same, equivalent or similar purpose, unless expressly stated otherwise. Thus, unless expressly stated otherwise, each feature disclosed is one example only of a generic series of equivalent or similar features.

## Claims

1. A dispensing device (1, 20) for at least one air treatment agent, the dispensing device comprising:
a housing (2, 27) having one or more walls to define an interior adapted to receive at least one removable container (3, 22) of air treatment agent at least partially therein; and within said housing the device comprises:
an airborne agent detector means (7, 30) operable to detect airborne agents in the air, wherein said means are provided with at least one aperture to the exterior of the device to permit, in use, air from outside of the device to enter said airborne agent detector means;
receiving means (4, 34) for receiving said at least one container of air treatment agent;
emanation means (5, 29) adapted, in use, to emanate the air treatment agent from the device through one or more exit orifices in the housing;
control means (6) in communication with the emanation means and in communication with said airborne agent detector means;
**characterised in that** said airborne agent detector means is substantially completely isolated from any fluid present in the interior of the housing such that any fluid present in the interior of the housing is substantially completely prevented from passing through said one or more housing walls to be detectable by the airborne agent detector means.

2. A dispensing device according to claim 1, wherein the at least one aperture for the airborne agent detector means is spaced away from the one or more exit orifices.

3. A dispensing device according to claim 1 or claim 2, wherein the at least one aperture is located in a housing wall that is substantially perpendicular with a housing wall of the exit orifice(s).

4. A dispensing device according to claim 1 or claim 2, wherein the at least one aperture is located in a housing wall that is substantially opposite to a housing wall of the exit orifice(s).

5. A dispensing device according to any preceding claim, wherein the housing is provided with a concave recess in a wall thereof that extends into the interior of the housing, said recess being sized to receive the airborne agent detector means therein.

6. A dispensing device according to claim 5, wherein the concave recess is provided with a cover that is sized to fill the recess to and substantially follow the shape and/or contour of the housing wall, and wherein said cover comprises the at least one aperture.

7. A dispensing device according to any preceding claim, wherein the aperture(s) are provided with a filter membrane to prevent or substantially prevent particulate contamination of the airborne agent detector means whilst allowing gas diffusion therethrough.

8. A dispensing device according to any preceding claim, wherein the device is provided with a movable closure means which is configured to close the aperture(s) to the airborne agent detector means to the outside environment shortly before the device emanates an air treatment agent and remain closed for a period after emanation has occurred.

9. A dispensing device according to any preceding claim, wherein the housing is provided with an outwardly extending protrusion adjacent to the aperture(s) communicating with the airborne agent detector means in order to prevent an emanated air treatment agent from entering the aperture(s).

10. A dispensing device according to any preceding claim, wherein the airborne agent detector means comprises at least one odour sensor means.

11. A dispensing device according to claim 10, wherein the odour sensor means comprises one or more metal oxide semiconductor sensors and/or one or more metal oxide sensors.

12. A method of improving the sensitivity of a dispensing device with an airborne agent detector mean for at least one air treatment agent, wherein the dispensing device (1, 20) comprises:
a housing (2, 27) having one or more walls to define an interior adapted to receive at least one removable container (3, 22) of air treatment agent at least partially therein; and within said housing the device comprises:
an airborne agent detector means (7, 30) operable to detect airborne agents in the air, wherein said means are provided with at least one aperture to the exterior of the device to permit, in use, air from outside of the device to enter said airborne agent detector means;
receiving means (4, 34) for receiving said at least one container of air treatment agent;
emanation means (5, 29) adapted, in use, to emanate the air treatment agent from the device through one or more exit orifices in the housing;
control means (6) in communication with the emanation means and in communication with said airborne agent detector means;
and wherein said airborne agent detector means is substantially completely isolated from any fluid present in the interior of the housing such that any fluid present in the interior of the housing is substantially completely prevented from passing through said one or more housing walls to be detectable by the airborne agent detector means;
and wherein the method comprises the steps of:
loading a container of air treatment agent into said housing and engaging the receiving means;
placing the device in an operational mode wherein the emanation means engage with the container of air treatment agent to cause a quantity of air treatment agent to emanate from the device;
**characterised in that** any air treatment agent that remains in the device after the emanation thereof is not capable to emanate from within the device to come into contact with the airborne agent detector which is substantially completely isolated therefrom.

## Patentansprüche

1. Spendervorrichtung (1, 20) für mindestens einen Luftbehandlungsstoff, wobei die Spendervorrichtung umfasst:
ein Gehäuse (2, 27) mit einer oder mehreren Wänden zum Festlegen eines Inneren, das dazu beschaffen ist, mindestens einen entfernbaren Behälter (3, 22) mit Luftbehandlungsstoff mindestens teilweise darin aufzunehmen; und wobei die Vorrichtung innerhalb des Gehäuses umfasst:
ein Erfassungsmittel (7, 30) für Schwebstoff, das zum Erfassen von Schwebstoffen in der Luft betreibbar ist, wobei die Mittel mit mindestens einer Öffnung zur Außenseite der Vorrichtung versehen sind, um zu ermöglichen, dass bei Verwendung Luft von außerhalb der Vorrichtung in das Erfassungsmittel für Schwebstoff eintritt;
Aufnahmemittel (4, 34) zum Aufnehmen des mindestens einen Behälters mit Luftbehandlungsstoff;
Ausströmungsmittel (5, 29), die bei Verwendung dazu beschaffen sind, den Luftbehandlungsstoff durch eine oder mehrere Austrittsöffnungen in dem Gehäuse aus der Vorrichtung ausströmen zu lassen;
Steuermittel (6) in Kommunikation mit den Ausströmungsmitteln und in Kommunikation mit dem Erfassungsmittel für Schwebstoff;
**dadurch gekennzeichnet, dass** das Erfassungsmittel für Schwebstoff im Wesentlichen vollständig von jeglichem im Inneren des Gehäuses vorhandenen Fluid isoliert ist, sodass jegliches im Inneren des Gehäuses vorhandene Fluid im Wesentlichen vollständig daran gehindert wird, durch die eine oder die mehreren Gehäusewände zu dringen, um von dem Erfassungsmittel für Schwebstoff erfassbar zu sein.

2. Spendervorrichtung nach Anspruch 1, wobei die mindestens eine Öffnung für das Erfassungsmittel für Schwebstoff von der einen oder den mehreren Austrittsöffnungen beabstandet ist.

3. Spendervorrichtung nach Anspruch 1 oder Anspruch 2, wobei sich die mindestens eine Öffnung in einer Gehäusewand befindet, die im Wesentlichen senkrecht zu einer Gehäusewand der Ausgangsöffnung(en) ist.

4. Spendervorrichtung nach Anspruch 1 oder Anspruch 2, wobei sich die mindestens eine Öffnung in einer Gehäusewand befindet, die einer Gehäusewand der Ausgangsöffnung(en) im Wesentlichen gegenüberliegt.

5. Spendervorrichtung nach einem der vorhergehenden Ansprüche, wobei das Gehäuse mit einer konkaven Vertiefung in einer Wand davon versehen ist, die sich in das Innere des Gehäuses erstreckt, wobei die Vertiefung so bemessen ist, dass sie das Erfassungsmittel für Schwebstoff darin aufnimmt.

6. Spendervorrichtung nach Anspruch 5, wobei die konkave Vertiefung mit einer Abdeckung versehen ist, die so bemessen ist, dass sie die Vertiefung bis zur Form und/oder Kontur der Gehäusewand ausfüllt und dieser im Wesentlichen folgt, und wobei die Abdeckung die mindestens eine Öffnung umfasst.

7. Spendervorrichtung nach einem der vorhergehenden Ansprüche, wobei die Öffnung(en) mit einer Filtermembran versehen sind, um eine Partikelverunreinigung des Erfassungsmittels für Schwebstoff zu verhindern oder im Wesentlichen zu verhindern, während eine Gasdiffusion durch dieses möglich ist.

8. Spendervorrichtung nach einem der vorhergehenden Ansprüche, wobei die Vorrichtung mit einem beweglichen Schließmittel versehen ist, das konfiguriert ist, um die Öffnung(en) zum Erfassungsmittel für Schwebstoff gegenüber der Außenumgebung kurz vor dem Ausströmen eines Luftbehandlungsstoffes durch die Vorrichtung zu schließen und das Schließen für einen Zeitraum nach Durchführen der Ausströmung aufrechtzuerhalten.

9. Spendervorrichtung nach einem der vorhergehenden Ansprüche, wobei das Gehäuse mit einem sich nach außen erstreckenden Vorsprung neben der/den Öffnung(en) versehen ist, der mit dem Erfassungsmittel für Schwebstoff in Kommunikation ist, um zu verhindern, dass ein ausströmender Luftbehandlungsstoff in die Öffnung(en) eintritt.

10. Spendervorrichtung nach einem der vorhergehenden Ansprüche, wobei das Erfassungsmittel für Schwebstoff mindestens ein Geruchssensormittel umfasst.

11. Spendervorrichtung nach Anspruch 10, wobei das Geruchssensormittel einen oder mehrere Metalloxidhalbleitersensoren und/oder einen oder mehrere Metalloxidsensoren umfasst.

12. Verfahren zum Verbessern der Empfindlichkeit einer Spendervorrichtung mit einem Erfassungsmittel für Schwebstoff für mindestens einen Luftbehandlungsstoff, wobei die Spendervorrichtung (1, 20) umfasst:
ein Gehäuse (2, 27) mit einer oder mehreren Wänden zum Festlegen eines Inneren, das dazu beschaffen ist, mindestens einen entfernbaren Behälter (3, 22) mit Luftbehandlungsstoff mindestens teilweise darin aufzunehmen; und wobei die Vorrichtung innerhalb des Gehäuses umfasst:
ein Erfassungsmittel (7, 30) für Schwebstoff, das zum Erfassen von Schwebstoffen in der Luft betreibbar ist, wobei die Mittel mit mindestens einer Öffnung zur Außenseite der Vorrichtung versehen sind, um zu ermöglichen, dass bei Verwendung Luft von außerhalb der Vorrichtung in das Erfassungsmittel für Schwebstoff eintritt;
Aufnahmemittel (4, 34) zum Aufnehmen des mindestens einen Behälters mit Luftbehandlungsstoff;
Ausströmungsmittel (5, 29), die bei Verwendung dazu beschaffen sind, den Luftbehandlungsstoff durch eine oder mehrere Austrittsöffnungen in dem Gehäuse aus der Vorrichtung ausströmen zu lassen;
Steuermittel (6) in Kommunikation mit den Ausströmungsmitteln und in Kommunikation mit dem Erfassungsmittel für Schwebstoff;
und wobei das Erfassungsmittel für Schwebstoff im Wesentlichen vollständig von jeglichem im Inneren des Gehäuses vorhandenen Fluid isoliert ist, sodass jegliches im Inneren des Gehäuses vorhandene Fluid im Wesentlichen vollständig daran gehindert wird, durch die eine oder die mehreren Gehäusewände zu dringen, um von dem Erfassungsmittel für Schwebstoff erfassbar zu sein;
und wobei das Verfahren die Schritte umfasst:
Laden eines Behälters mit Luftbehandlungsstoff in das Gehäuse und Eingreifen in die Aufnahmemittel;
Versetzen der Vorrichtung in einen Betriebsmodus, wobei die Ausströmungsmittel in den Behälter mit Luftbehandlungsstoff eingreifen, um zu bewirken, dass eine Menge von Luftbehandlungsstoff aus der Vorrichtung ausströmt;
**dadurch gekennzeichnet, dass** jeglicher Luftbehandlungsstoff, der nach dessen Ausströmung in der Vorrichtung verbleibt, nicht in der Lage ist, aus der Vorrichtung auszuströmen, um mit dem Erfassungsmittel für Schwebstoff in Kontakt zu kommen, das im Wesentlichen vollständig davon isoliert ist.

## Revendications

1. Dispositif de distribution (1, 20) pour au moins un agent de traitement de l'air, le dispositif de distribution comprenant :
un boîtier (2, 27) ayant une ou plusieurs parois pour définir un intérieur adapté pour recevoir au moins partiellement en son sein un conteneur amovible (3, 22) d'agent de traitement de l'air ;
et à l'intérieur dudit boîtier, le dispositif comprend :
un moyen de détection d'agents aéroportés (7, 30) pouvant fonctionner pour détecter des agents aéroportés dans l'air, dans lequel ledit moyens est pourvu d'au moins une ouverture vers l'extérieur du dispositif pour permettre, en cours d'utilisation, à l'air provenant de l'extérieur du dispositif d'entrer dans lesdits moyens de détection d'agents aéroportés ;
des moyens de réception (4, 34) pour recevoir ledit au moins un conteneur d'agent de traitement de l'air ;
des moyens d'émission (5, 29) adaptés, en cours d'utilisation, pour émettre l'agent de traitement de l'air du dispositif à travers un ou plusieurs orifices de sortie dans le boîtier ;
des moyens de commande (6) en communication avec les moyens d'émission et en communication avec ledit moyen de détection d'agents aéroportés ;
**caractérisé en ce que** ledit moyen de détection d'agents aéroportés est sensiblement complètement isolé de tout fluide présent à l'intérieur du boîtier de telle sorte que tout fluide présent à l'intérieur du boîtier ne puisse sensiblement complètement pas passer à travers la ou lesdites parois de boîtier pour être détectable par le moyen de détection d'agents aéroportés.

2. Dispositif de distribution selon la revendication 1, dans lequel l'au moins une ouverture pour le moyen de détection d'agents aéroportés est éloignée du ou des orifices de sortie.

3. Dispositif de distribution selon la revendication 1 ou la revendication 2, dans lequel l'au moins une ouverture est située dans une paroi de boîtier qui est sensiblement perpendiculaire à une paroi de boîtier du ou des orifices de sortie.

4. Dispositif de distribution selon la revendication 1 ou la revendication 2, dans lequel l'au moins une ouverture est située dans une paroi de boîtier qui est sensiblement opposée à une paroi de boîtier du ou des orifices de sortie.

5. Dispositif de distribution selon une quelconque revendication précédente, dans lequel le boîtier est pourvu d'un évidement concave dans une de ses parois qui s'étend à l'intérieur du boîtier, ledit évidement étant dimensionné pour recevoir en son sein le moyen de détection d'agents aéroportés.

6. Dispositif de distribution selon la revendication 5, dans lequel l'évidement concave est pourvu d'un couvercle qui est dimensionné pour remplir l'évidement et suivre sensiblement la forme et/ou un contour de la paroi de boîtier, et dans lequel ledit couvercle comprend l'au moins une ouverture.

7. Dispositif de distribution selon une quelconque revendication précédente, dans lequel la ou les ouvertures sont pourvues d'une membrane filtrante pour empêcher ou empêcher sensiblement la contamination particulaire du moyen de détection d'agents aéroportés tout en permettant la diffusion de gaz à travers celle-ci.

8. Dispositif de distribution selon une quelconque revendication précédente, dans lequel le dispositif est pourvu d'un moyen de fermeture mobile qui est configuré pour fermer la ou les ouvertures du moyen de détection d'agents aéroportés à l'environnement extérieur peu de temps avant que le dispositif n'émette un agent de traitement de l'air et reste fermé pendant un certain délai après l'émission.

9. Dispositif de distribution selon une quelconque revendication précédente, dans lequel le boîtier est pourvu d'une saillie s'étendant vers l'extérieur adjacente à la ou aux ouvertures communiquant avec le moyen de détection d'agents aéroportés afin d'empêcher un agent de traitement de l'air émis d'entrer dans la ou les ouvertures.

10. Dispositif de distribution selon une quelconque revendication précédente, dans lequel le moyen de détection d'agents aéroportés comprend au moins un moyen de détection d'odeurs.

11. Dispositif de distribution selon la revendication 10, dans lequel le moyen de détection d'odeurs comprend un ou plusieurs détecteurs semi-conducteurs à oxyde métallique et/ou un ou plusieurs détecteurs à oxyde métallique.

12. Procédé d'amélioration de la sensibilité d'un dispositif de distribution comportant un moyen de détection d'agents aéroportés pour au moins un agent de traitement de l'air, dans lequel le dispositif de distribution (1, 20) comprend :
un boîtier (2, 27) ayant une ou plusieurs parois pour définir un intérieur adapté pour recevoir au moins partiellement en son sein un conteneur amovible (3, 22) d'agent de traitement de l'air ;
et à l'intérieur dudit boîtier, le dispositif comprend :
un moyen de détection d'agents aéroportés (7, 30) pouvant fonctionner pour détecter des agents aéroportés dans l'air, dans lequel ledit moyen est pourvu d'au moins une ouverture vers l'extérieur du dispositif pour permettre, en cours d'utilisation, à l'air provenant de l'extérieur du dispositif d'entrer dans lesdits moyens de détection d'agents aéroportés ;
des moyens de réception (4, 34) pour recevoir ledit au moins un conteneur d'agent de traitement de l'air ;
des moyens d'émission (5, 29) adaptés, en cours d'utilisation, pour émettre l'agent de traitement de l'air du dispositif à travers un ou plusieurs orifices de sortie dans le boîtier ;
des moyens de commande (6) en communication avec les moyens d'émission et en communication avec ledit moyen de détection d'agents aéroportés ;
et dans lequel ledit moyen de détection d'agents aéroportés est sensiblement complètement isolé de tout fluide présent à l'intérieur du boîtier de telle sorte que tout fluide présent à l'intérieur du boîtier ne puisse sensiblement complètement pas traverser la ou lesdites parois de boîtier pour être détectable par le moyen de détection d'agent aéroporté ;
et dans lequel le procédé comprend les étapes :
de chargement d'un conteneur d'agent de traitement de l'air dans ledit boîtier et de mise en prise des moyens de réception ;
de positionnement du dispositif dans un mode de fonctionnement dans lequel les moyens d'émission sont en prise avec le conteneur d'agent de traitement de l'air pour provoquer l'émission d'une quantité d'agent de traitement de l'air à partir du dispositif ;
**caractérisé en ce que** tout agent de traitement de l'air qui reste dans le dispositif après son émission n'est pas en mesure de sortir de l'intérieur du dispositif pour entrer en contact avec le détecteur d'agents aéroportés qui est sensiblement complètement isolé de celui-ci.
